# EUROPEAN PATENT SPECIFICATION

(11) **EP 1 161 417 B1**
(45) Date of publication and mention of the grant of the patent: **03.12.2003**
(21) Application number: 00908883.2
(22) Date of filing: 13.03.2000
(51) Int. Cl.: C07C 323/60, C07D 277/74, A61K 31/277, A61K 31/428, A61P 35/02

(54) **METHODS AND COMPOSITIONS FOR TREATING LEUKEMIA**
VERFAHREN UND ZUSAMMENSETZUNGEN ZUR BEHANDLUNG VON LEUKÄMIE
PROCEDES ET COMPOSITIONS DESTINES AU TRAITEMENT DE LEUCEMIES

(30) Priority: 12.03.1999 CA 2265396
(43) Date of publication of application: 12.12.2001
(73) Proprietor: HSC Research and Development Limited Partnership, Toronto, Ontario M5G 1X8 (CA); Yissum Research Development Company, of The Hebrew University of Jerusalem, 91390 Jerusalem (IL)
(72) Inventor: ROIFMAN, Chaim, M., North York, Ontario M2R 1A4 (CA); GAZIT, Aviv, Jerusalem (IL); LEVITZKI, Alexander, 92542 Jerusalem (IL)
(74) Representative: Tanner, James Percival
(86) International application number: CA0000266
(87) International publication number: WO00055128

(56) References cited:
- WO-A-94/26260
- US-A- 5 773 476
- A. GAZIT, ET AL.: "Tyrphostins. 2. Heterocyclic and alpha-substituted benzylidenemalononitrile tyrphostins as potent inhibitors of EGF receptor and ErbB2/neu tyrosine kinases" JOURNAL OF MEDICINAL CHEMISTRY, vol. 34, no. 6, 1 June 1991 (1991-06-01), pages 1896-1907, XP000472938 American Chemical Society, Washington, DC, US ISSN: 0022-2623

## Description

### Field of the Invention

This invention relates to tyrphostins or benzylidene malononitrile compounds which are useful as antiproliferative pharmaceuticals for treating a variety of cell proliferative disorders.

### Background of the Invention

A number of tyrphostins or benzylidene malononitrile derivatives have been described which are tyrosine kinase inhibitors and are effective to inhibit cell proliferation, for example in human leukemia (United States Patents Nos. 5,217,999 and 5,773,476).

### Summary of the Invention

The present invention provides a new group of tyrphostins or benzylidene malononitrile derivatives of improved effectiveness as inhibitors of cell growth.

In accordance with one embodiment, the compounds of the invention have the general formula: 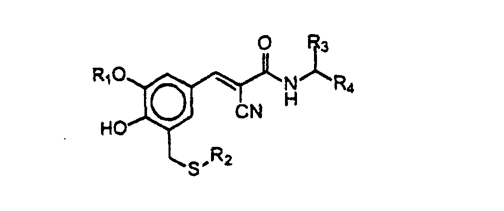 wherein
R₁ is H or C1 to C3 alkyl;
R₂ is aryl or -(CH₂)ₙ- aryl and n is 1 to 4;
R₃ is H or CH₃; and
R₄ is substituted or unsubstituted phenyl, pyridyl, thiophene, furan, indole, pyrrole, thiazole, imidazole.

In accordance with a further embodiment, the compounds have the general formula 1 above, wherein
R₁ is H, methyl or ethyl;
R₂ is phenyl, benzyl, -(CH₂)₂-phenyl, -(CH₂)₃-phenyl or 2-thiobenzothiazole;
R₃ is H; and
R₄ is phenyl.

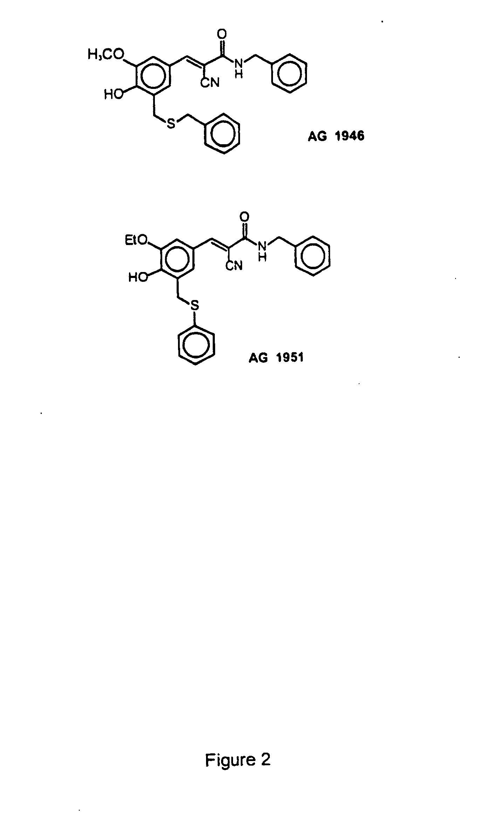

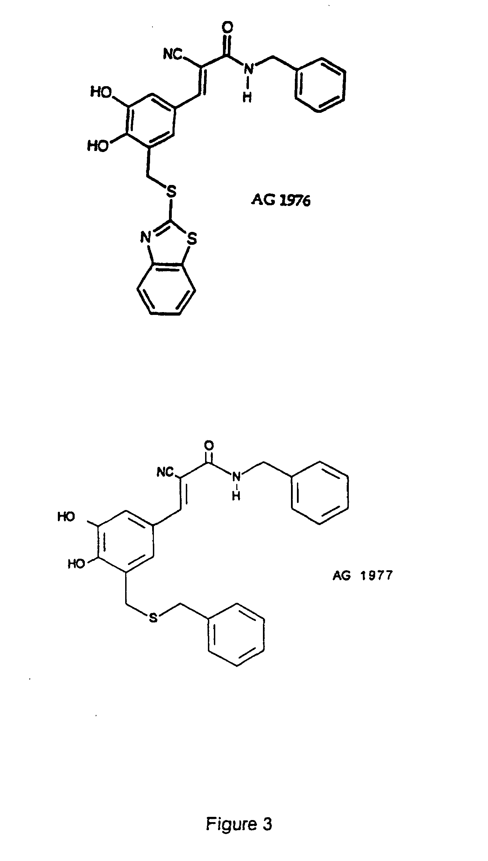

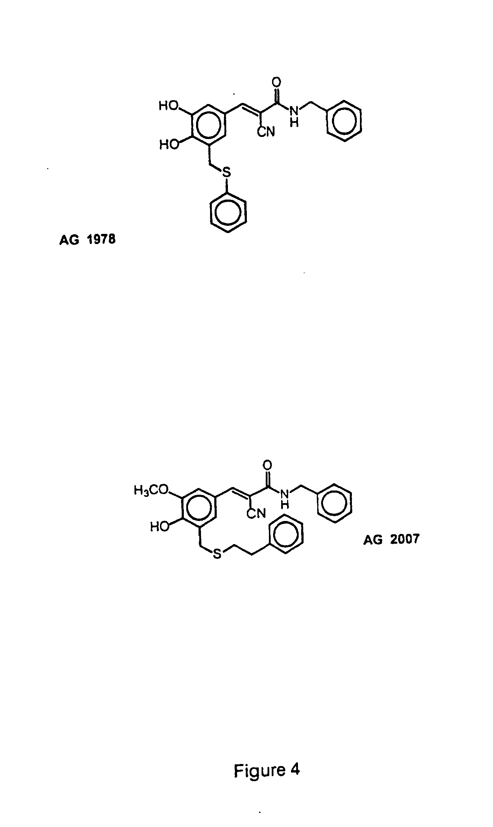

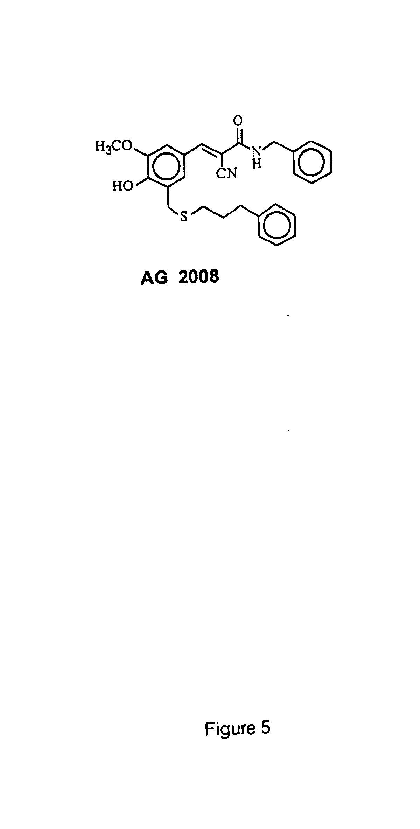

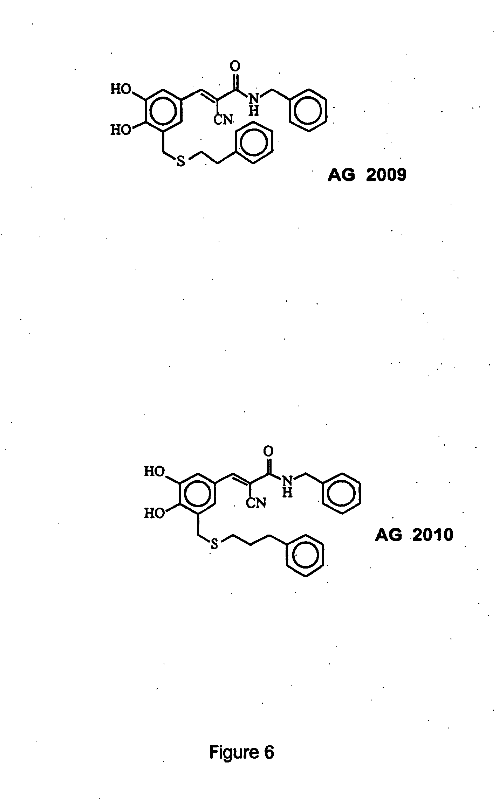

In accordance with a preferred embodiment, the compounds are those shown in Figures 2 to 6.

In accordance with a further embodiment, the invention provides a pharmaceutical composition comprising as active ingredient a compound of formula 1 above.

In accordance with a further embodiment, the invention provides a pharmaceutical composition comprising as active ingredient one of the compounds shown in Figures 2 to 6.

In accordance with a further embodiment, the invention provides a compound of formula 1 above for treating a neoplastic disorder, preferably acute lymphoblastic leukemia (ALL), in a mammal.

In accordance with a further embodiment, the invention provides at least one of the compounds shown in Figures 2 to 6 for treating a neoplastic disorder, preferably acute lymphoblastic leukemia (ALL), in a mammal.

In accordance with a further embodiment, the invention provides the use of a compound of formula 1 above in the preparation of a medicament for treating a neoplastic disorder, preferably acute lymphoblastic leukemia (ALL), in a mammal.

In accordance with a further embodiment, the invention provides at least one of the compounds shown in Figures 2 to 6 in the preparation of a medicament for treating a neoplastic disorder, preferably acute lymphoblastic leukemia (ALL), in a mammal.

In accordance with a further embodiment, the invention provides a compound of formula 1 above for treating a cell proliferative disorder in a mammal.

In accordance with a further embodiment, the invention provides at least one of the compounds shown in Figures 2 to 6 for treating a cell proliferative disorder in a mammal.

In accordance with a further embodiment, the invention provides the use of a compound of formula 1 above in the preparation of a medicament for treating a cell proliferative disorder in a mammal.

In accordance with a further embodiment, the invention provides at least one of the compounds shown in Figures 2 to 6 in the preparation of a medicament for treating a cell proliferative disorder in a mammal.

### Summary of the Drawings

Figure 1 shows in schematic form a process for synthesising the compounds of the invention. R₁ is C1 to C3 alkyl; R₂ is aryl or -(CH₂)ₙ-aryl, where n is 1 to 4; R₃ is H or CH₃; and R₄ is substituted or unsubstituted phenyl, pyridyl, thiophene, furan, indole, pyrrole, thiazole or imidazole.
Figures 2 to 6 show some examples of the compounds of the invention.
Figure 7 shows the inhibitory effect (expressed as colony formation/1.5 x 10⁵ cells) of several compounds of the invention on growth of G2 ALL cells.
Figure 8 shows the inhibitory effect of several compounds of the invention on growth of G2 ALL cells.
Figures 9, 10, 11 and 12 show the effect of compounds AG 1977, AG 1978, AG 2009 and AG 2010 respectively on the growth of normal BM cells, as indicated by three different assays.
Figure 13 shows inhibition of G2 ALL cells by various concentrations of the compounds of the invention.
Figure 14 shows a dose response curve of G2 ALL cell inhibition and AG 2009 concentration.
Figure 15 shows inhibition C1 ALL cells by various concentrations of AG 1977 and 1978.
Figure 16 shows inhibition of A1 ALL cells by various concentrations of the compounds of the invention.
Figure 17 shows inhibition of blast cells by several compounds of the invention.

### Detailed Description of the Invention

A number of tyrphostins, including the compound α-cyano-3,4-dihydroxy-cinnamal benzylamide (AG 490), have previously been shown to inhibit the growth of leukemia cells, and to be useful as active ingredients in pharmaceutical compositions for treating leukemia.

The present inventors have found that a new group of substituted benzylidene malononitriles have unexpectedly improved efficacy for treatment of cell proliferative diseases. For example, the inventors have shown that the tyrphostins described herein gave complete suppression of proliferation of human acute lymphoblastic leukemia (ALL) cells and of pre-B ALL blast cells without significantly affecting normal bone marrow cells, as described in the examples.

The compounds of the present invention have the formula: 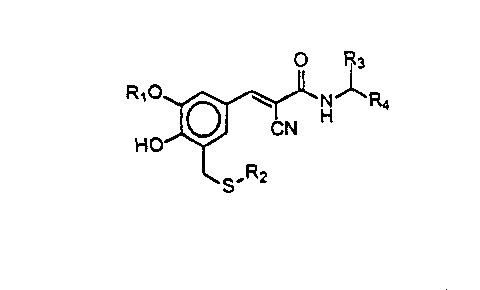 wherein
R₁ is H or C1 to C3 alkyl;
R₂ is aryl or -(CH₂)ₙ- aryl and n is 1 to 4;
R₃ is H or CH₃; and
R₄ is substituted or unsubstituted phenyl, pyridyl, thiophene, furan, indole, pyrrole, thiazole or imidazole.

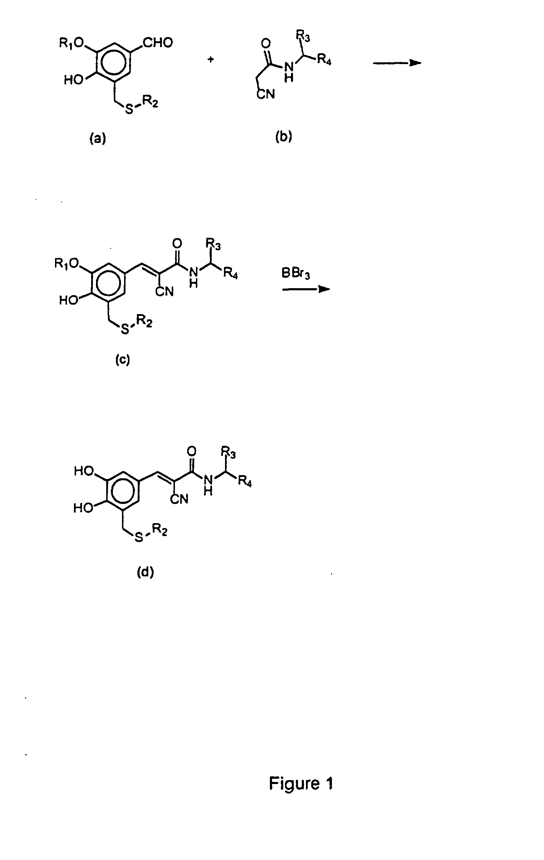

The compounds of the invention are prepared by the process shown schematically in Figure 1. The required aldehydes (a) are available commercially or can be synthesised as previously described (Gazit et al., (1993), J. Med. Chem., v. 36, p. 3556). Benzyl cyano acetamide (b) is synthesised as described previously (Gazit et al., (1991), J. Med. Chem., v. 34, p. 1896).

A preferred group of compounds are the compounds shown in Figures 2 to 6.

The compounds of the invention may be used to treat a variety of neoplastic disorders, including leukemia, lymphomas, metastatic carcinomas and other forms of cancer. Leukemias which may be treated include B-lineage Acute Lymphoblastic Leukemia (ALL), such as the aggressive Philadelphia⁺ leukemia, and acute myelocytic leukemia and juvenile myelo monocytic leukemia; lymphomas which may be treated include B-lineage Burkitt's lymphoma and Non-Hodgkin's lymphomas, such as the Ki-1 positive anaplastic large cell lymphomas.

The compounds of the invention may also be used to reduce or inhibit cell growth in a variety of cell proliferative disorders such as inflammatory disorders, allergic disorders, autoimmune diseases and graft rejection situations in which cell growth suppression, and preferably T cell growth suppression, is desired.

The compounds of the invention may also be used to inhibit the activity of Jak2 kinase. They may therefore be used to treat any disorder associated with increased or undesired Jak2 kinase activity.

The compounds of this invention may be used in the form of the free base, in the form of salts and as hydrates. All forms are within the scope of the invention. Acid addition salts may be formed and provide a more convenient form for use; in practice, use of the salt form inherently amounts to use of the base form. The acids which can be used to prepare the acid addition salts include preferably those which produce, when combined with the free base, pharmaceutically acceptable salts, that is, salts whose anions are non-toxic to the animal organism in pharmaceutical doses of the salts, so that the beneficial properties inherent in the free base are not vitiated by side effects ascribable to the anions. Although pharmaceutically acceptable salts of the basic compounds are preferred, all acid addition salts are useful as sources of the free base form even if the particular salt per se is desired only as an intermediate product as, for example, when the salt is formed only for the purposes of purification and identification, or when it is used as an intermediate in preparing a pharmaceutically acceptable salt by ion exchange procedures.

Pharmaceutically acceptable salts within the scope of the invention include those derived from the following acids; mineral acids such as hydrochloric acid, sulfuric acid, phosphoric acid and sulfamic acid: and organic acids such as acetic acid, citric acid, lactic acid, tartaric acid, malonic acid, methanesulfonic acid, ethanesulfonic acid, benzenesulfonic acid, p-toluenesulfonic acid, cyclohexylsulfamic acid, quinic acid, and the like.

Compounds may be examined for their efficacy in inhibiting cell growth in cell proliferation assays such as those described herein.

In accordance with the the invention, the described tyrphostins may be administered to a leukemia patient in a variety of forms depending on the selected route of administration, as will be understood by those skilled in the art. The compositions of the invention may be administered orally or parenterally, the latter route including intravenous and subcutaneous administration. Parenteral administration may be by continuous infusion over a selected period of time.

The active compound may be orally administered, for example. with an inert diluent or with an assimilable edible carrier, or it may be enclosed in hard or soft shell gelatin capsules, or it may be compressed into tablets, or it may be incorporated directly with the food of the diet. For oral therapeutic administration, the active compound may be incorporated with excipient and used in the form of ingestible tablets, buccal tablets, troches, capsules, elixirs. suspensions, syrups, wafers, and the like.

The active compound may also be administered parenterally or intraperitoneally. Solutions of the active compound as a free base or pharmacologically acceptable salt can be prepared in water suitably mixed with a surfactant such as hydroxypropylcellulose. Dispersion can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersion and sterile powders for the extemporaneous preparation of sterile injectable solutions or dispersions. In all cases the form must be sterile and must be fluid to the extent that easy syringability exists.

The therapeutic compounds of this invention may be administered to a mammal alone or in combination with pharmaceutically acceptable carriers, as noted above, the proportion of which is determined by the solubility and chemical nature of the compound, chosen route of administration and standard pharmaceutical practice.

Tyrphostins are administered initially in a suitable dosage to provide a blood level of about 10 µM. The dosage may be adjusted as required, depending on the clinical response.

An alternate therapeutic approach is to obtain bone marrow or peripheral blood cells containing stem cells from patients with leukemia or lymphoma and to treat these cells ex vivo with a tyrphostin of the invention to purge or kill leukemia or lymphoma cells present with minimal inhibition of normal stem cells. The treated cells are washed to remove excess tyrphostin and returned to the patient.

For such ex vivo treatment of cells over a short period, for example around 5 hours, higher doses of tyrphostin may be used than for long term in vivo therapy; for example, concentrations of 50µM or higher may be used.

### Examples

The Examples are described for the purposes of illustration and are not intended to limit the scope of the invention.

### Example 1 - Synthesis of Compounds

The compounds of the invention were synthesised generally by the process shown schematically in Figure 1.

### Compound AG 1946: N-benzyl-2-cyano-3-(4'-hydroxy-3'-methoxy-5'-methylene thiobenzyl phenyl) acrylamide

R₁ = CH₃, R₂= benzyl, R₃= H, R₄ = phenyl
(a) 207 mg 0.72mM 4-hydroxy-3-methoxy-5-methylene thiobenzyl benzaldehyde, 130 mg 0.75 mM N-benzyl cyano acetamide and 10 mg β-alanine in 25 mL ethanol were refluxed 3 hours. Evaporation and trituration with dichloromethane-hexane gave 305 mg yellow-green solid, 95% yield, mp-135°.
NMR (acetone d₆) δ 8.17(1H,s,vinyl), 7.70(1H,d,J=2.1 Hz),
7.57(1H,d,J=2.1 Hz), 7.3(10H,m), 4.60(2H,s), 3.93(3H,s), 3.78(2H,s), 3.74(2H,s).

### Compound AG 1977: N-benzyl-2-cyano-3-(3',4'-dihydroxy-5'-methylene thiobenzyl phenyl) acrylamide

R₁ = H, R₂ = benzyl, R₃ = H, R₄ = phenyl
(b) To 450 mg of product from (a) in 30 mL dichloromethane was added 0.5 mL BBr₃. After stirring 1 hour at room temperature, water was added and the reaction extracted with ethyl acetate. Evaporation and trituration with dichloromethane-hexane gave 340 mg, 78% yield. yellow solid, mp-195°.
NMR (acetone d₆) δ 8.08(1 H,s,vinyl), 7.62(1 H,d,J=2.2 Hz), 7.3(11H.m). 4.58(2H,s), 3.78(2H,s), 3.75(2H,s).
MS m/e-430(M⁺,16%), 175(100%).

### Compound AG 1951: N-benzyl-2-cyano-3-(3'-ethoxy-4'-hydroxy-5'-methylene thiophenyl phenyl) acrylamide

R₁ = CH₂CH₃, R₂ = phenyl, R₃ = H, R₄ = phenyl
(c) 500 mg 1.74mM 3-ethoxy-4-hydroxy-5-methylene thiophenyl benzaldehyde, 310 mg 1.78 mM N-benzyl cyano acetamide and 25 mg β-alanine in 40 mL ethanol were refluxed 4 hours. Evaporation and trituration with hexane gave 730 mg yellow solid, 95% yield, mp-108°. NMR (acetone d₆) δ 8.10(1H,s,vinyl), 7.70(1H,d,J=2.2 Hz), 7.53(1H,d,J=2.2 Hz), 7.3(10H,m), 4.58(2H,d,J=6.0 Hz), 4.26(2H,s), 4.18(2H,q,J=7.0 Hz), 1.42(3H,t,J=7.0 Hz).

### Compound AG 1978: N-benzyl-2-cyano-3-(3',4-dihydroxy-5'-methylene thiophenyl phenyl) acrylamide

R₁ = H, R₂ = phenyl, R₃ = H, R₄ = phenyl
(d) To 200 mg of product from (c) in 30 mL dichloromethane was added 0.4 mL BBr₃. After stirring 1 hour at room temperature water was added and the reaction extracted with ethyl acetate. Evaporation and trituration with dichloromethane-hexane gave 91 mg, 47% yield, yellow solid, mp-175°.
NMR (acetone d₆) δ 8.01 (1H.s,vinyl), 7.63(1H,d,J=2.2 Hz), 7.3(11H,m), 4.58(2H,s), 4.26(2H,s).
MS m/e- 416(M⁺, 16%), 309(12), 263(32), 196(37), 175(100%).

### Compound AG 2007: N-benzyl-2-cyano-3-(4'-hydroxy-3'-methoxy-5'-(methylene thioethyl phenyl) phenyl) acrylamide

R₁ = CH₃, R₂ = CH₂CH₂Ph, R₃ = H, R₄ = phenyl
(e) 400 mg 1.3 mM 4-hydroxy-3-methoxy-5-methylene thiophenethyl benzaldehyde, 240 mg 1.38 mM N-benzyl cyano acetamide and 20 mg β-alanine in 40 mL ethanol were refluxed 4 hours. Evaporation and trituration with dichloromethane- hexane gave 450 mg yellow solid, 88% yield, mp-102°.
NMR (CDC1₃) δ 8.25(1H,s,vinyl), 7.62(1H,d,J=2.2 Hz), 7.3(11H,m), 4.61(2H,d,J=6.0 Hz), 3.96(3H,s), 3.81(2H,s), 2.80(4H,m).

### Compound AG 2009: N-benzyl-2-cyano-3-(3',4'-dihydroxy-5'-(methylene thioethyl phenyl) phenyl) acrylamide

R₁ = H, R₂ = CH₂CH₂Ph, R₃ = H, R₄ = phenyl
(f) To 320 mg of product from (e) in 25 mL dichloromethane was added 0.3 mL BBr₃. After stirring 1 hour at room temperature water was added and the reaction extracted with ethyl acetate. Evaporation and trituration with dichloromethane-hexane gave 110 mg, 35% yield, yellow solid, mp-153°.
NMR (acetone d₆) δ 8.09(1H,s,vinyl), 7.63(1H,d,J=2.2 Hz), 7.3(11H,m), 4.58(2H,s), 3.85(2H,s), 2.80(4H,m).

### Compound AG 2008: N-benzyl-2-cyano-3-(4'-hydroxy-3'-methoxy-5'-(methylene thiopropyl phenyl) phenyl) acrylamide

R₁ = CH₃, R₂ = CH₂CH₂CH₂Ph, R₃ = H, R₄ = phenyl
(g) 800 mg 2.5 mM 4-hydroxy-3-methoxy-5-methylene thiopropylphenyl benzaldehyde, 430 mg, 2.5 mM N-benzyl cyano acetamide and 20 mg β-alanine in 40 mL ethanol were refluxed 4 hours. Evaporation and trituration with Cc1₄ gave 760 mg yellow solid, 63% yield, mp-78°.
NMR (CDC1₃) δ 8.25(1H,s,vinyl), 7.62(1H,d,J=2.2 Hz), 7.3(11H,m), 4.61(2H,d,J=6.0 Hz), 3.96(3H,s), 3.81(2H,s), 2.69(2H,t,J=6.0 Hz). 2.50(2H,t,J=6.0 Hz), 1.90(2H,quint.,J=6.0 Hz).

### Compound AG 2010: N-benzyl-2-cyano-3-(3',4'-dihydroxy-5'-(methylene thiopropyl phenyl) phenyl) acrylamide

R₁ = H, R₂ = CH₂CH₂CH₂Ph, R₃ = H, R₄ = phenyl
(h) To 660 mg of product from (g) in 25 mL dichloromethane was added 0.6 mL BBr₃. After stirring 1 hour at room temperature water was added and the reaction extracted with ethyl acetate. Evaporation and trituration with dichloromethane-hexane gave 610 mg, 95% yield. yellow solid, mp-138°.
NMR (acetone d₆) δ 8.17(1H,s,vinyl), 7.63(1H,d,J=2.2 Hz), 7.3(11H,m), 4.58(2H,s), 3.80(2H,s), 2.69(2H,t,J=6.0 Hz), 2.52(2H,t,J=6.0 Hz), 1.90(2H,quint.,J=6.0 Hz).

### Compound AG 1976: N-benzyl-2-cyano-3-(3',4'-dihydroxy-5'-methylene (2'-thiobenzothiazole) phenyl) acrylamide

R₁ = H, R₂ = 2-thiobenzothiazole, R₃ = H, R₄ = phenyl
(i) 330 mg, 1 mM, 4-hydroxy-3-methoxy-5-methylene (2-thiobenzothiazole) benzaldehyde, 180 mg, 1.03 mM, N-benzyl cyano acetamide and 15 mg β-alanine in 20 ml ethanol were refluxed 4 hours. Cooling and filtering gave 460 mg, 95% yield, yellow solid.
(j) To 200 mg, 0.4 mM, solid from (i) in 30 ml dichloromethane was added 0.4 ml BBr₃. After stirring 1 hour at room temperature, water and 3 ml HCl was added and the reaction extracted with ethyl acetate. Evaporation and trituration with dichloromethane-hexane gave 40 mg, 20% yield, bright yellow solid, mp-225%.
NMR Acetone d₆ δ 8.07(1H.s,vinyl), 7.95(2H,m), 7.6 7.1(9H,m), 4.60(2H,s), 4.48(2H,d.J=5.9 Hz).

### Example 2 - Inhibition of Colony Formation - Acute Lymphoblastic Leukemia (ALL) Cell Lines

Inhibition of colony formation was studied by methods described previously (Kamel-Reid et al., (1992), Leukemia, v. 6, pp. 8-17; Meydan et al., (1996), Nature, v. 379, pp. 645-648).

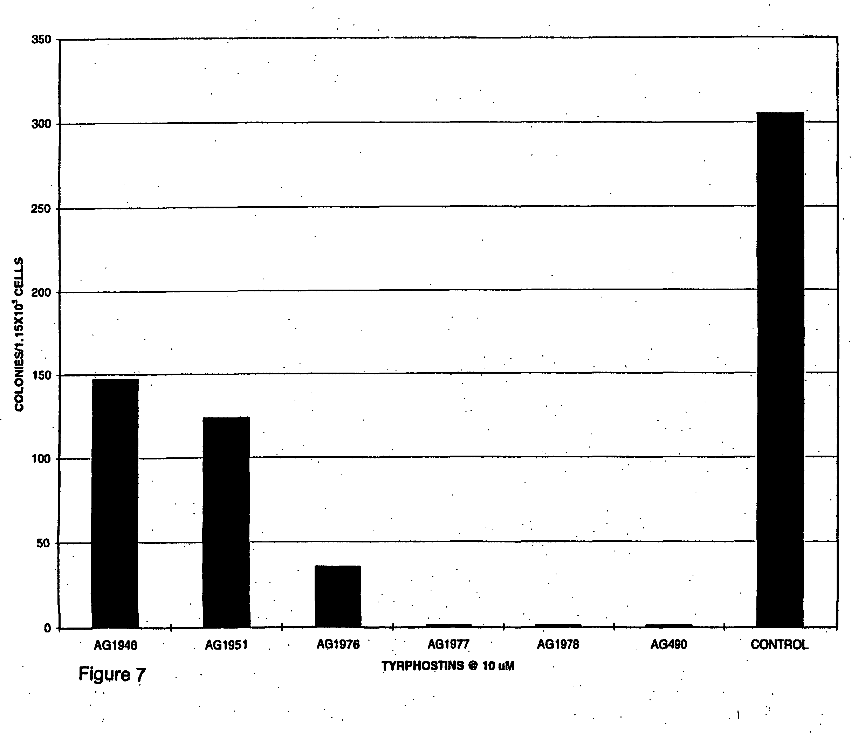

ALL cell lines A1 (at 8x10⁵ cells/ml), C1 (at 4x10⁴ cells/ml) and G2 (at 1.15x10⁶ cells/ml) were plated in 1 ml volumes, in the absence of exogenous growth factors, into 35 mm petri dishes (Nunc, Gibco) containing alpha MEM (Gibco) plus 10% FCS (Cansera Rexdale, Ont.) in 0.9% (vol/vol) methylcellulose (Fluka, Switzerland). Cultures were set up at 37°C with 5% CO₂ in a humidified atmosphere and 10 uM of a selected tyrphostin was added. Colonies consisting of more than 20 cells were counted at 12 days (A1), 5 days (C1) and 14 days (G2) using an inverted microscope. The results with G2 are shown in Figure 7. Similar results were obtained with A1 and C1.

### Example 3 - Effect on Bone Marrow Cells

Compounds showing inhibition of ALL colony formation were examined for their effect on normal bone marrow cells using a modified CFU-GEMM clonogenic assay.

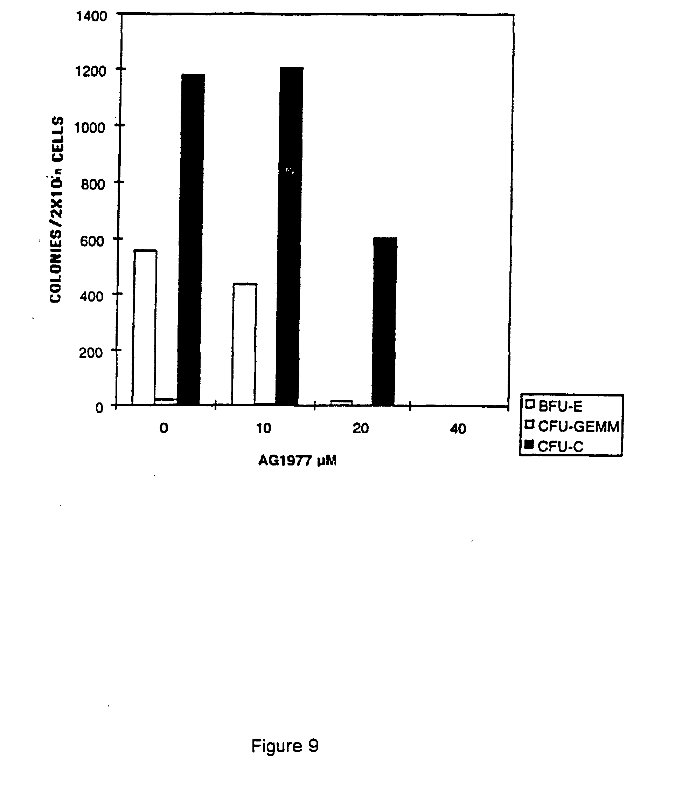

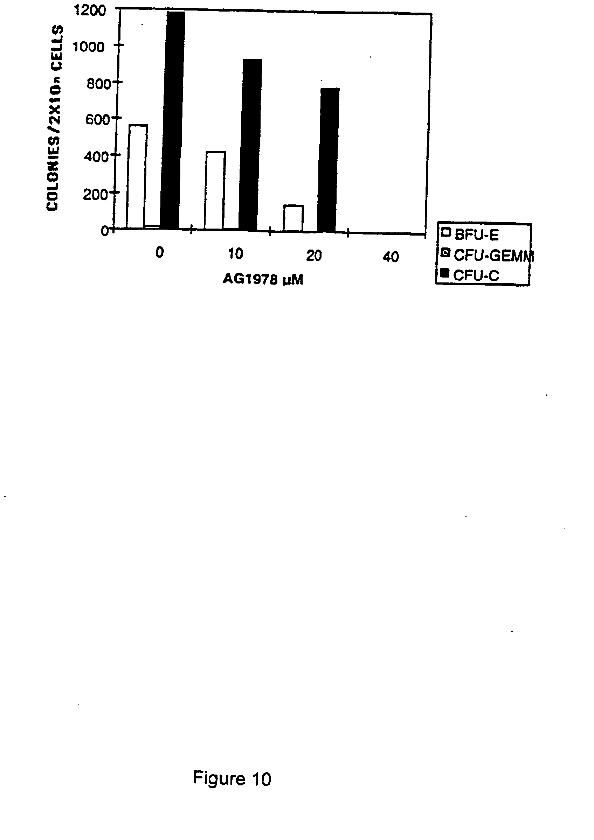

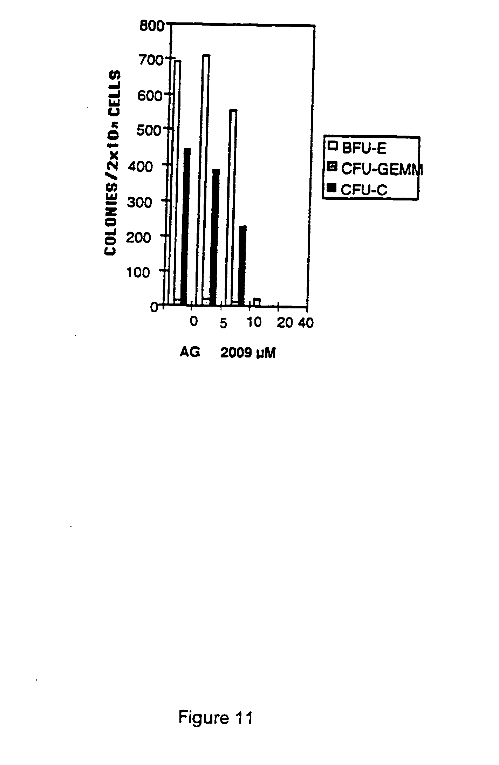

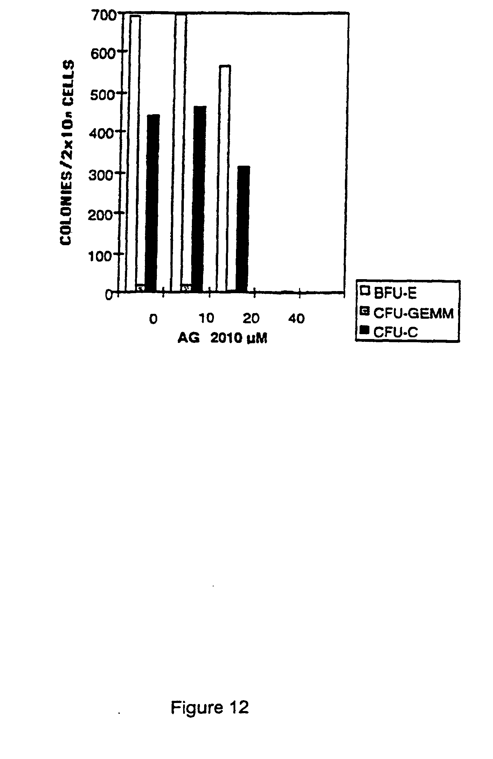

The assay was performed according to Fauser and Messner (1978), Blood, v. 52, pp. 1243-8, and Messner and Fausser (1980), Blut, v. 41, pp. 327-333, with some variations. In brief, heparinized bone marrow cells were layered over Percoll (Pharmacia Fine Chemical. Piscataway N.J.) at a density of 1.077 gm/ml and centrifuged at 400 g at 4°C for 10 min. to remove neutrophils and RBCs. The fractionated bone marrow cells at 2x10⁵ cells/ml were cultured in IMDM (OCI, Toronto) containing 0.9% (vol/vol) methylcellulose supplemented with 30% FCS (Cansera Rexdale, Ont.) or normal human plasma, a cocktail of cytokines consisting of G-CSF (10 ng/ml. Amgen), IL-3 (40 U/ml, Immunex), MGF (50 ng/ml, Immunex), Erythropoietin (2u/ml, Epprex) or TPO (10 ng/ml, Amgen) and 5x10⁻⁵ 2-mercaptoethanol. The culture mixture was plated in 1 ml volumes into 35 mm petri dishes and incubated at 37°C with 5% CO₂ in a humidified atmosphere with concentrations of tyrphostin up to 40 µm. The results are shown in Figures 9 to 12.

The BFU-E's (erythroid colonies) and the CFU-GEMM (mixed colonies) exhibited inhibition at and above 25µM (Fig. not shown), while the CFU-C's (granulocytes, monocytes and macrophages) showed a dramatic increase of colony proliferation peaking at 25µM and a reduction by 50µM (Fig. not shown). AG 2010 showed significant inhibition at 40µM, while the remaining compounds showed mild to significant inhibition of erythroid and mixed colonies followed by the myeloid population at 20µM.

### Example 4 - Inhibition of ALL Cells

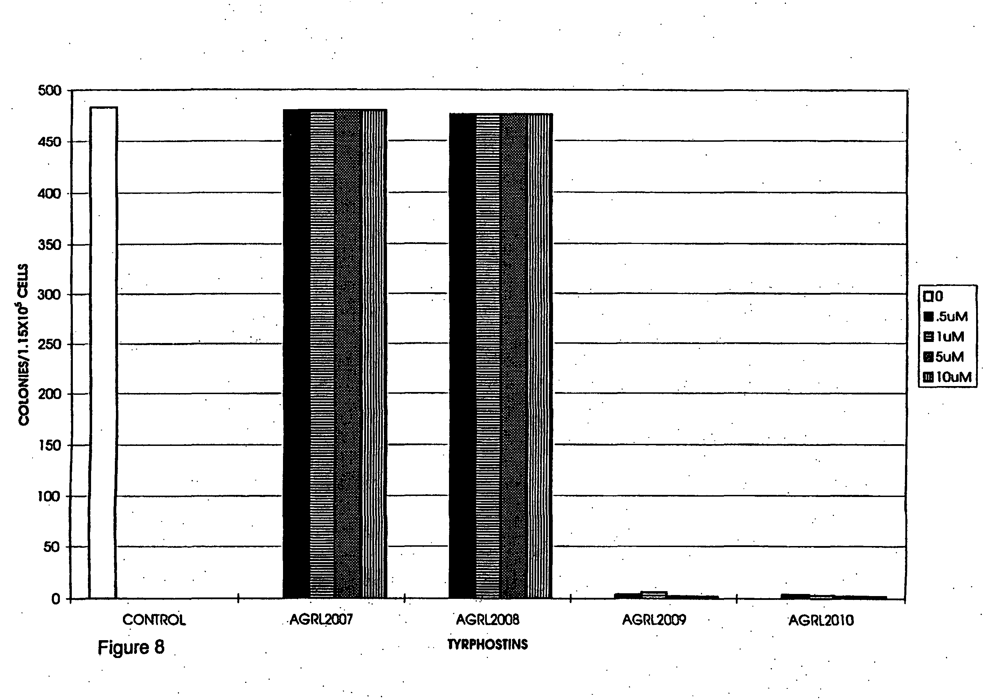

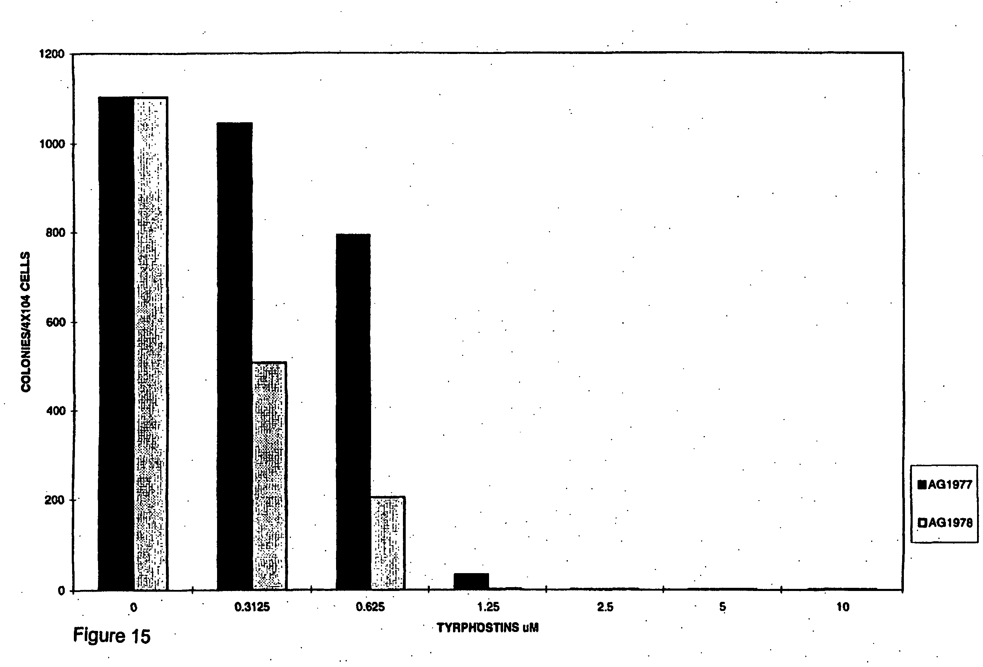

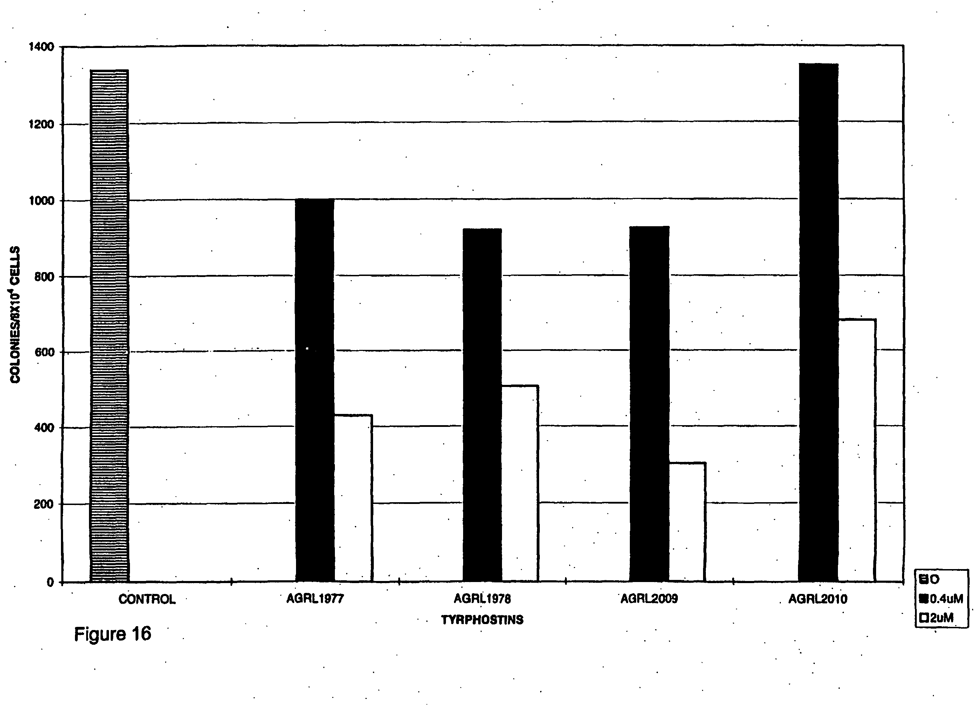

Various concentrations of tyrphostins were tested for inhibition of ALL cells in the clonogenic assay described in Example 2. Compounds AG 1977. 1978, 2007, 2008, 2009 and 2010 were tested against ALL cell lines A1, C1 and G2 in doses ranging from nanomolar to micromolar values. The results are shown in Figures 8, 13, 15 and 16.

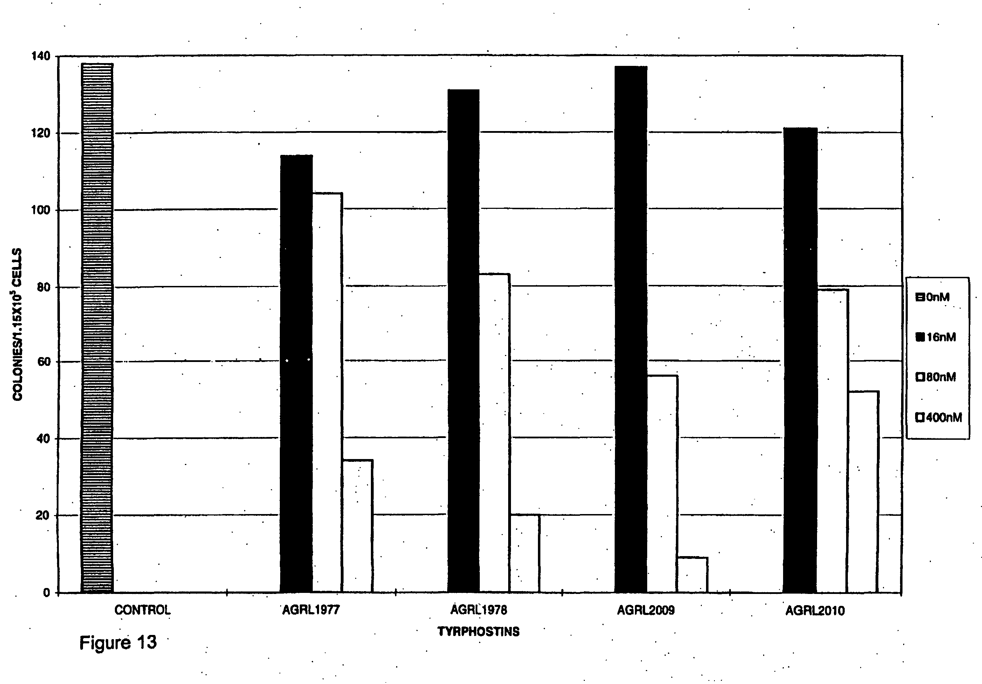

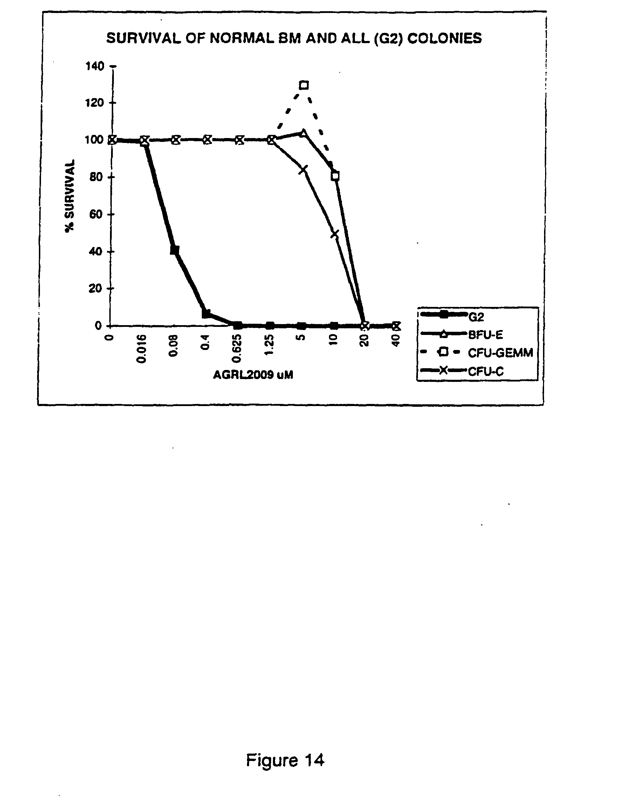

AG 2009 demonstrated the most potent clonogenic inhibition, in a dose responsive manner, against G2 cells (Fig. 13). It showed a greater than 50% inhibition at a dose of 16nM and a differential therapeutic index of greater than 2 logs in a survival curve (Fig. 14) of normal BM and G2 colonies.

### Example 5 - Inhibition of Blast Cells

The compounds were further tested in an ALL blast colony assay, against bone marrow samples from two patients with pre -B ALL phenotype based on their FAB classification.

The ALL blast colony assay was performed as described previously (Estrov, Z. et al., (1988), Cancer Res., v. 48, p. 5901) with some modifications. Briefly, heparinized bone marrow cells were layered over Percoll (density 1.077 g/l; Pharmacia Fine Chemicals Piscataway, N.J.) and centrifuged (400g) for 10 minutes at 4°C to remove neutrophils and RBC's. The collected interphase fraction was further enriched for Iymphoblasts before plating by using a magnetic cell separator, mini MACS, with separation column (Miltenyi Biotec Inc., 1250 Oakmead Park, Sunnyvale, Ca). With this procedure, ALL blasts were specifically isolated from the marrow mononuclear cell fraction using directly labelled MACS CD19 Microbeads-monoclonal anti-human CD19 (Mouse IgG1, Kappa- Miltenyi Biotec Inc.) and/or indirect magnetic cell labeling using primary biotinylated antibody-(mouse anti-human CD10 monoclonal antibody, Caltag Laboratires, Ca.) and Streptavidin Microbeads (Miltenyi Biotech Inc.). The resulting cell population was composed of 99% lymphoblasts. The positively sorted cells were then cultured at 2x10⁵ cells/ml in alpha MEM (GIBCO) containing 0.9% (vol/vol) methylcellulose supplemented with 10% FCS (Cansera, Rexdale, Ont.). Irradiated autologous leukemic blasts were used as feeder cells (at 3x10⁵ cells/ml). Cytokines, normally used, were deleted so that only spontaneous proliferation was evident.

The culture mixture was plated into 35mm petri dishes (Nunc. GIBCO) containing 1ml volumes and incubated at 37°C with 5% CO₂ in a humidified atmosphere. Colonies containing more than 20 cells were scored, using inverted microscope, at 5-7 days.

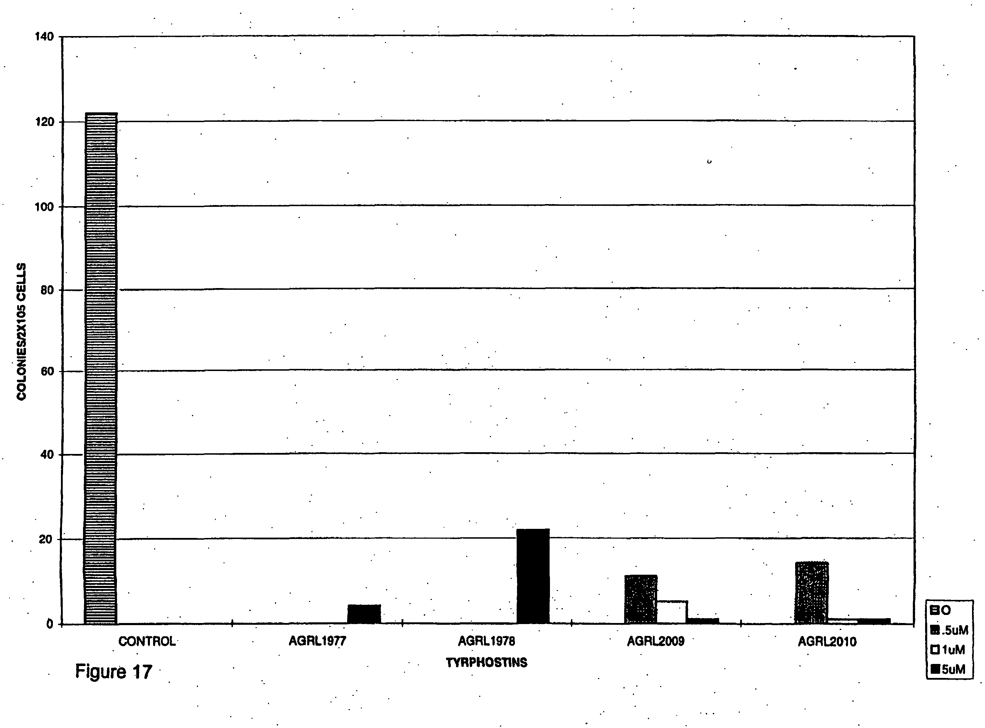

In both cases, significant inhibition of blast colonies was observed (See for example, Figure 17).

### Example 6 - Inhibition of Jak2 Kinase Activity

Compounds chosen on the basis of their ability to inhibit the growth (colony formation) of the pre-B leukemia cell line G2 were tested for inhibition of Jak2 kinase.

Jak2 kinase was immunoprecipitated from a 1% Triton-X100 lysate of 10x10⁶ G2 cells. An *in vitro* kinase assay was performed on the immunoprecipitated Jak2 in the presence or absence of varying concentrations of the compounds AG 1977, AG 1978, AG 2009 and AG 2010.

Stock solutions of 100mM tyrphostin were made in 100% DMSO and further dilutions made in 10% DMSO. Control kinase assays carried out in the presence of DMSO concentrations of 5-30% alone were unaffected by its presence.

Initial experiments were done with tyrphostin concentrations of 0.1, 1.0 and 10µM. These concentrations had no affect on the kinase activity of Jak2. Using higher concentrations, 5, 25 and 50µM, inhibition could be seen, as shown in Table 1 below. These results were obtained by scanning autoradiographs of the kinase assays.

**TABLE 1**

| %inhibition of kinase activity | | | |
|---|---|---|---|
| | | Concentration | |
| Tyrphostin | 5µM | 25µM | 50µM |
| AG 1977 | 23 | 14 | 15 |
| AG 1978 | 10 | 13 | 46 |
| AG 2009 | 0 | 0 | 8 |
| AG 2010 | 5 | 5 | 39 |

## Claims

1. A compound of the general formula: 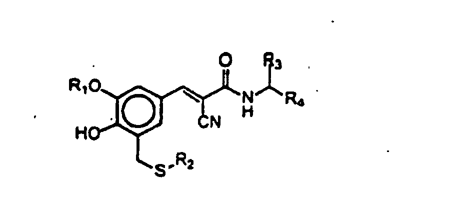 wherein
R₁ is H or C1 to C3 alkyl;
R₂ is aryl or -(CH₂)ₙ- aryl and n is 1 to 4;
R₃ is H or CH₃; and
R₄ is substituted or unsubstituted phenyl, pyridyl, thiophene, furan, indole, pyrrole, thiazole, or imidazole,
or a salt or hydrate thereof.

2. A compound in accordance with claim 1 wherein
R₁ is H, methyl or ethyl;
R₂ is phenyl, benzyl, -(CH₂)₂-phenyl, -(CH₂)₃-phenyl or 2-thiobenzothiazole;
R₃ is H; and
R₄ is phenyl.

3. A compound in accordance with claim 1 wherein the compound is N-benzyl-2-cyano-3-(4'-hydroxy-3'-methoxy-5'-methylene thiobenzyl phenyl) acrylamide.

4. A compound in accordance with claim 1 wherein the compound is N-benzyl-2-cyano-3-(3'-ethoxy-4'-hydroxy-5'-methylene thiophenyl phenyl) acrylamide.

5. A compound in accordance with claim1 wherein the compound is N-benzyl-2-cyano-3-(3',4'-dihydroxy-5'-methylene (2'-thiobenzothiazole) phenyl) acrylamide.

6. A compound in accordance with claim 1 wherein the compound is N-benzyl-2-cyano-3-(3',4'-dihydroxy-5'-methylene thiobenzyl phenyl) acrylamide.

7. A compound in accordance with claim 1 wherein the compound is N-benzyl-2-cyano-3-(3',4'-dihydroxy-5'-methylene thiophenyl phenyl) acrylamide.

8. A compound in accordance with claim 1 wherein the compound is N-benzyl-2-cyano-3-(4'-hydroxy-3'-methoxy-5'-(methylene thioethyl phenyl) phenyl) acrylamide.

9. A compound in accordance with claim 1 wherein the compound is N-benzyl-2-cyano-3-(4'-hydroxy-3'-methoxy-5'-(methylene thiopropyl phenyl) phenyl) acrylamide.

10. A compound in accordance with claim 1 wherein the compound is N-benzyl-2-cyano-3-(3',4'-dihydroxy-5'-(methylene thioethyl phenyl) phenyl) acrylamide.

11. A compound in accordance with claim 1 wherein the compound is N-benzyl-2-cyano-3-(3',4'-dihydroxy-5'-(methylene thiopropyl phenyl) phenyl) acrylamide.

12. A pharmaceutical composition comprising a compound of the formula: 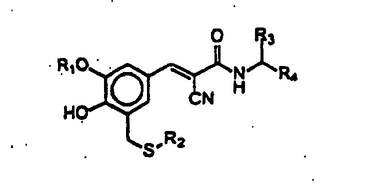 wherein
R₁ is H or C1 to C3 alkyl;
R₂ is aryl or -(CH₂)ₙ- aryl and n is 1 to 4;
R₃ is H or CH₃; and
R₄ is substituted or unsubstituted phenyl, pyridyl, thiophene, furan, indole, pyrrole, thiazole or imidazole,
or salt or hydrate thereof
and a pharmaceutically acceptable carrier

13. A pharmaceutical composition in accordance with claim 12 comprising a compound of formula I, wherein
R₁ is H, methyl or ethyl;
R₂ is phenyl, benzyl, -(CH₂)₂-phenyl, -(CH₂)₂-phenyl or 2-thiobenzothiazole;
R₃ is H; and
R₄ is phenyl.

14. A pharmaceutical composition in accordance with claim 12 wherein the compound is N-benzyl-2-cyano-3-(4'-hydroxy-3'-methoxy-5'-methylene thiobenzyl phenyl) acrylamide.

15. A pharmaceutical composition in accordance with claim 12 wherein the compound is N-benzyl-2-cyano-3-(3'-ethoxy-4'-hydroxy-5'-methylene thiophenyl phenyl) acrylamide.

16. A pharmaceutical composition in accordance with claim 12 wherein the compound is N-benzyl-2-cyano-3-(3',4'-dihydroxy-5'-methylene (2'-thiobenzothiazole) phenyl) acrylamide

17. A pharmaceutical composition in accordance with claim 12 wherein the compound is N-benzyl-2-cyano-3-(3',4'-dihydroxy-5'-methylene thiobenzyl phenyl) acrylamide.

18. A pharmaceutical composition in accordance with claim 12 wherein the compound is N-benzyl-2-cyano-3-(3',4'-dihydroxy-5'-methylene thiophenyl phenyl) acrylamide.

19. A pharmaceutical composition in accordance with claim 12 wherein the compound is N-benzyl-2-cyano-3-(4'-hydroxy-3'-methoxy-5'-(methylene thioethyl phenyl) phenyl) acrylamide.

20. A pharmaceutical composition in accordance with claim 12 wherein the compound is N-benzyl-2-cyano-3-(4'-hydroxy-3'-methoxy-5'-(methylene thiopropyl phenyl) phenyl) acrylamide.

21. A pharmaceutical composition in accordance with claim 12 wherein the compound is N-benzyl-2-cyano-3-(3',4'-dihydroxy-5'-(methylene thioethyl phenyl) phenyl) acrylamide.

22. A pharmaceutical composition in accordance with claim 12 wherein the compound is N-benzyl-2-cyano-3-(3',4'-dihydroxy-5'-(methylene thiopropyl phenyl) phenyl) acrylamide.

23. A compound as claimed in any of claims 1 to 11 for treating a neoplastic disorder in a mammal.

24. A compound in accordance with claim 23, wherein the neoplastic disorder is a lymphoma, a leukemia or a metastatic carcinoma.

25. A compound in accordance with claim 23 wherein the neoplastic disorder is Acute Lymphoblastic Leukemia.

26. Use of a compound as claimed in any of claims 1 to 11 in the preparation of a medicament for treating a neoplastic disorder in a mammal.

27. Use in accordance with claim 25 wherein the neoplastic disorder is a lymphoma, a leukemia or a metastatic carcinoma.

28. Use in accordance with claim 25 wherein the neoplastic disorder is Acute Lymphoblastic Leukemia.

29. A compound as claimed in any of claims 1 to 11 for treating a cell proliferative disorder in a mammal.

30. A compound in accordance with claim 29 wherein the cell proliferative disorder is selected from the group consisting of an inflammatory disorder, an allergic disorder, an autoimmune disease or graft rejection.

31. Use of a compound as claimed in any of claims 1 to 11 in the preparation of a medicament for treating a cell proliferative disorder in a mammal.

32. Use in accordance with claim 31 wherein the cell proliferative disorder is selected from the group consisting of an inflammatory disorder, an allergic disorder, an autoimmune disease or graft rejection.

## Patentansprüche

1. Verbindung der allgemeinen Formel 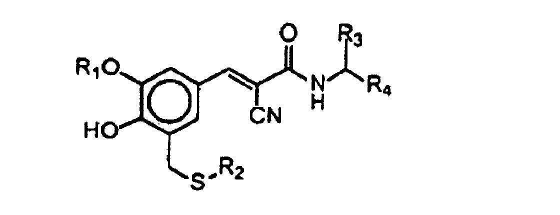 wobei
R₁ H oder C1- bis C3-Alkyl ist,
R₂ Aryl oder -(CH₂)ₙ-Aryl und n 1 bis 4 ist,
R₃ H oder CH₃ ist und
R₄ substituiertes oder unsubstituiertes Phenyl, Pyridyl, Thiophen, Furan, Indol, Pyrrol, Thiazol oder Imidazol ist,
oder ein Salz oder Hydrat davon.

2. Verbindung nach Anspruch 1, wobei
R₁ H, Methyl oder Ethyl ist,
R₂ Phenyl, Benzyl, -(CH₂)₂-Phenyl, -(CH₂)₃-Phenyl oder 2-Thiobenzothiazol ist,
R₃ H ist und
R₄ Phenyl ist.

3. Verbindung nach Anspruch 1, wobei die Verbindung N-Benzyl-2-cyano-3-(4'-hydroxy-3'methoxy-5'-methylenthiobenzylphenyl)-acrylamid ist.

4. Verbindung nach Anspruch 1, wobei die Verbindung N-Benzyl-2-cyano-3-(3'-ethoxy-4'hydroxy-5'-methylenthiophenylphenyl)-acrylamid ist.

5. Verbindung nach Anspruch 1, wobei die Verbindung N-Benzyl-2-cyano-3-(3',4'-dihydroxy-5'methylen-(2'-thiobenzothiazol)-phenyl)-acrylamid ist.

6. Verbindung nach Anspruch 1, wobei die Verbindung N-Benzyl-2-cyano-3-(3',4'-dihydroxy-5'methylenthiobenzylphenyl)-acrylamid ist.

7. Verbindung nach Anspruch 1, wobei die Verbindung N-Benzyl-2-cyano-3-(3',4'-dihydroxy-5'methylenthiophenylphenyl)-acrylamid ist.

8. Verbindung nach Anspruch 1, wobei die Verbindung N-Benzyl-2-cyano-3-(4'-hydroxy-3'methoxy-5'-(methylenthioethylphenyl)-phenyl)-acrylamid ist.

9. Verbindung nach Anspruch 1, wobei die Verbindung N-Benzyl-2-cyano-3-(4'-hydroxy-3'methoxy-5'-(methylenthiopropylphenyl)-phenyl)-acrylamid ist.

10. Verbindung nach Anspruch 1, wobei die Verbindung N-Benzyl-2-cyano-3-(3',4'-dihydroxy-5'-(methylenthioethylphenyl)-phenyl)-acrylamid ist.

11. Verbindung nach Anspruch 1, wobei die Verbindung N-Benzyl-2-cyano-3-(3',4'-dihydroxy-5'-(methylenthiopropylphenyl)-phenyl)-acrylamid ist.

12. Pharmazeutische Zusammensetzung mit einer Verbindung der Formel: 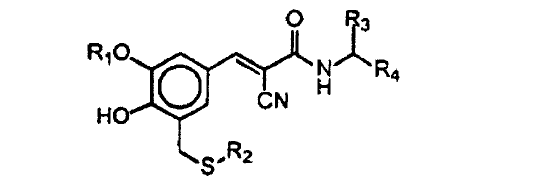 wobei
R₁ H oder C1- bis C3-Alkyl ist,
R₂ Aryl oder -(CH₂)ₙ-Aryl und n 1 bis 4 ist,
R₃ H oder CH₃ ist und
R₄ substituiertes oder unsubstituiertes Phenyl, Pyridyl, Thiophen, Furan, Indol, Pyrrol, Thiazol oder Imidazol ist,
oder einem Salz oder Hydrat davon
und einem pharmazeutisch verträglichen Träger.

13. Pharmazeutische Zusammensetzung nach Anspruch 12 mit einer Verbindung der Formel I, wobei
R₁ H, Methyl oder Ethyl ist,
R₂ Phenyl, Benzyl, -(CH₂)₂-Phenyl, -(CH₂)₃-Phenyl oder 2-Thiobenzothiazol ist,
R₃ H ist und
R₄ Phenyl ist.

14. Pharmazeutische Zusammensetzung nach Anspruch 12, wobei die Verbindung N-Benzyl-2-cyano-3-(4'-hydroxy-3'-methoxy-5'-methylenthiobenzylphenyl)-acrylamid ist.

15. Pharmazeutische Zusammensetzung nach Anspruch 12, wobei die Verbindung N-Benzyl-2-cyano-3-(3'-ethoxy-4'-hydroxy-5'-methylenthiophenylphenyl)-acrylamid ist.

16. Pharmazeutische Zusammensetzung nach Anspruch 12, wobei die Verbindung N-Benzyl-2-cyano-3-(3',4'-dihydroxy-5'-methylen-(2'-thiobenzothiazol)-phenyl)-acrylamid ist.

17. Pharmazeutische Zusammensetzung nach Anspruch 12, wobei die Verbindung N-Benzyl-2-cyano-3-(3',4'-dihydroxy-5'-methylenthiobenzylphenyl)-acrylamid ist.

18. Pharmazeutische Zusammensetzung nach Anspruch 12, wobei die Verbindung N-Benzyl-2-cyano-3-(3',4'-dihydroxy-5'-methylenthiophenylphenyl)-acrylamid ist.

19. Pharmazeutische Zusammensetzung nach Anspruch 12, wobei die Verbindung N-Benzyl-2-cyano-3-(4'-hydroxy-3'-methoxy-5'-(methylenthioethylphenyl)-phenyl)-acrylamid ist.

20. Pharmazeutische Zusammensetzung nach Anspruch 12, wobei die Verbindung N-Benzyl-2-cyano-3-(4'-hydroxy-3'-methoxy-5'-(methylenthiopropylphenyl)-phenyl)-acrylamid ist.

21. Pharmazeutische Zusammensetzung nach Anspruch 12, wobei die Verbindung N-Benzyl-2-cyano-3-(3',4'-dihydroxy-5'-(methylenthioethylphenyl)-phenyl)-acrylamid ist.

22. Pharmazeutische Zusammensetzung nach Anspruch 12, wobei die Verbindung N-Benzyl-2-cyano-3-(3',4'-dihydroxy-5'-(methylenthiopropylphenyl)-phenyl)-acrylamid ist.

23. Verbindung nach einem der Ansprüche 1 bis 11 für das Behandeln einer neoplastischen Erkrankung in einem Säuger.

24. Verbindung nach Anspruch 23, wobei die neoplastische Erkrankung ein Lymphom, eine Leukämie oder ein metastasenbildendes Karzinom ist.

25. Verbindung nach Anspruch 23, wobei die neoplastische Erkrankung akute lymphoblastische Leukämie ist.

26. Verwendung einer Verbindung nach einem der Ansprüche 1 bis 11 bei der Herstellung eines Medikaments für die Behandlung einer neoplastischen Erkrankung in einem Säuger.

27. Verwendung nach Anspruch 25, wobei die neoplastische Erkrankung ein Lymphom, eine Leukämie oder ein metastasenbildendes Karzinom ist.

28. Verwendung nach Anspruch 25, wobei die neoplastische Erkrankung akute lymphoblastische Leukämie ist.

29. Verbindung nach einem der Ansprüche 1 bis 11 für das Behandeln einer zell-proliferativen Erkrankung in einem Säuger.

30. Verbindung nach Anspruch 29, wobei die zell-proliferative Erkrankung aus der Gruppe, die aus einer inflammatorischen Erkrankung, einer allergischen Erkrankung, einer Autoimmunerkrankung oder Transplantatabstoßung besteht, ausgewählt ist.

31. Verwendung einer Verbindung nach einem der Ansprüche 1 bis 11 bei der Herstellung eines Medikaments für das Behandeln einer zell-proliferativen Erkrankung in einem Säuger.

32. Verwendung nach Anspruch 31, wobei die zell-proliferative Erkrankung aus der Gruppe, die aus einer inflammatorischen Erkrankung, einer allergischen Erkrankung, einer Autoimmunerkrankung oder Transplantatabstoßung besteht, ausgewählt ist.

## Revendications

1. Composé de formule générale : 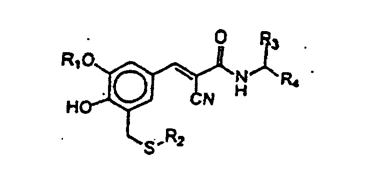 dans laquelle
R₁ représente H ou un groupe alkyle en C₁ à C₃ ;
R₂ représente un groupe aryle ou -(CH₂)ₙ-aryle et n a une valeur de 1 à 4 ;
R₃ représente H ou un groupe CH₃ ; et
R₄ représente un groupe, substitué ou non substitué, phényle, pyridyle, thiophène, furanne, indole, pyrrole, thiazole ou imidazole,
ou un de ses sels ou hydrates.

2. Composé suivant la revendication 1, dans lequel
R₁ représente H, un groupe méthyle ou éthyle ;
R₂ représente un groupe phényle, benzyle, -(CH₂)₂-phényle, -(CH₂)₃-phényle ou 2-thiobenzothiazole ;
R₃ représente H ; et
R₄ représente un groupe phényle.

3. Composé suivant la revendication 1, qui est le N-benzyl-2-cyano-3-(4'-hydroxy-3'-méthoxy-5'-méthylène-thiobenzylphényl)acrylamide.

4. Composé suivant la revendication 1, qui est le N-benzyl-2-cyano-3-(3'-éthoxy-4'-hydroxy-5'-méthylène-thiophényl-phényl)acrylamide.

5. Composé suivant la revendication 1, qui est le N-benzyl-2-cyano-3-(3',4'-dihydroxy-5'-méthylène(2'-thiobenzo-thiazole)-phényl)acrylamide.

6. Composé suivant la revendication 1, qui est le N-benzyl-2-cyano-3-(3',4'-dihydroxy-5'-méthylène-thiobenzylphényl)-acrylamide.

7. Composé suivant la revendication 1, qui est le N-benzyl-2-cyano-3-(3',4'-dihydroxy-5'-méthylène-thiophényl-phényl)acrylamide.

8. Composé suivant la revendication 1, qui est le N-benzyl-2-cyano-3-(4'-hydroxy-3'-méthoxy-5'-(méthylène-thioéthyl-phényl)phényl)acrylamide.

9. Composé suivant la revendication 1, qui est le N-benzyl-2-cyano-3-(4'-hydroxy-3'-méthoxy-5'-(méthylène-thiopropyl-phényl)phényl)acrylamide.

10. Composé suivant la revendication 1, qui est le N-benzyl-2-cyano-3-(3',4'-dihydroxy-5'-(méthylène-thioéthylphényl)-phényl)acrylamide.

11. Composé suivant la revendication 1, qui est le N-benzyl-2-cyano-3-(3',4'-dihydroxy-5'-(méthylène-thiopropyl-phényl)phényl)acrylamide.

12. Composition pharmaceutique comprenant un composé de formule 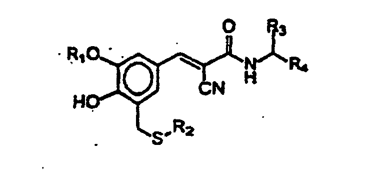 dans laquelle
R₁ représente H ou un groupe alkyle en C₁ à C₃ ;
R₂ représente un groupe aryle ou -(CH₂)ₙ-aryle et n a une valeur de 1 à 4 ;
R₃ représente H ou un groupe CH₃ ; et
R₄ représente un groupe, substitué ou non substitué, phényle, pyridyle, thiophène, furanne, indole, pyrrole, thiazole ou imidazole,
ou un de ses sels ou hydrates,
et un support pharmaceutiquement acceptable.

13. Composition pharmaceutique suivant la revendication 12, comprenant un composé de formule I, dans laquelle
R₁ représente H, un groupe méthyle ou éthyle ; R₂ représente un groupe phényle, benzyle, -(CH₂)₂-phényle, -(CH₂)₃-phényle ou 2-thiobenzothiazole ;
R₃ représente H ; et
R₄ représente un groupe phényle.

14. Composition pharmaceutique suivant la revendication 12, dans laquelle le composé est le N-benzyl-2-cyano-3-(4'-hydroxy-3'-méthoxy-5'-méthylène-thiobenzylphényl)acrylamide.

15. Composition pharmaceutique suivant la revendication 12, dans laquelle le composé est le N-benzyl-2-cyano-3-(3'-éthoxy-4'-hydroxy-5'-méthylène-thiophénylphényl)acrylamide.

16. Composition pharmaceutique suivant la revendication 12, dans laquelle le composé est le N-benzyl-2-cyano-3-(3',4'-dihydroxy-5'-méthylène(2'-thiobenzothiazole)phényl)-acrylamide.

17. Composition pharmaceutique suivant la revendication 12, dans laquelle le composé est le N-benzyl-2-cyano-3-(3',4'-dihydroxy-5'-méthylène-thiobenzylphényl)acrylamide.

18. Composition pharmaceutique suivant la revendication 12, dans laquelle le composé est le N-benzyl-2-cyano-3-(3',4'-dihydroxy-5'-méthylène-thiophénylphényl)acrylamide.

19. Composition pharmaceutique suivant la revendication 12, dans laquelle le composé est le N-benzyl-2-cyano-3-(4'-hydroxy-3'-méthoxy-5'-(méthylène-thioéthylphényl)phényl)acrylamide.

20. Composition pharmaceutique suivant la revendication 12, dans laquelle le composé est le N-benzyl-2-cyano-3-(4'-hydroxy-3'-méthoxy-5'-(méthylène-thiopropylphényl)phényl)acrylamide.

21. Composition pharmaceutique suivant la revendication 12, dans laquelle le composé est le N-benzyl-2-cyano-3-(3',4'-dihydroxy-5'-(méthylène-thioéthylphényl)phényl)acrylamide.

22. Composition pharmaceutique suivant la revendication 12, dans laquelle le composé est le N-benzyl-2-cyano-3-(3',4'-dihydroxy-5'-(méthylène-thiopropylphényl)phényl)acrylamide.

23. Composé suivant l'une quelconque des revendications 1 à 11, pour le traitement d'un trouble néoplasique chez un mammifère.

24. Composé suivant la revendication 23, dans lequel le trouble néoplasique est un lymphome, une leucémie ou un carcinome métastatique.

25. Composé suivant la revendication 23, dans lequel le trouble néoplasique est la leucémie lymphoblastique aiguë.

26. Utilisation d'un composé suivant l'une quelconque des revendications 1 à 11 dans la préparation d'un médicament destiné au traitement d'un trouble néoplasique chez un mammifère.

27. Utilisation suivant la revendication 25, dans laquelle le trouble néoplasique est un lymphome, une leucémie ou un carcinome métastatique.

28. Utilisation suivant la revendication 25, dans laquelle le trouble néoplasique est la leucémie lymphoblastique aiguë.

29. Composé suivant l'une quelconque des revendications 1 à 11, pour le traitement d'un trouble prolifératif cellulaire chez un mammifère.

30. Composé suivant la revendication 29, dans lequel le trouble prolifératif cellulaire est choisi dans le groupe consistant en un trouble inflammatoire, un trouble allergique, une maladie auto-immune et le rejet d'un greffon.

31. Utilisation d'un composé suivant l'une quelconque des revendications 1 à 11, dans la préparation d'un médicament destiné au traitement d'un trouble prolifératif cellulaire chez un mammifère.

32. Utilisation suivant la revendication 31, dans laquelle le trouble prolifératif cellulaire est choisi dans le groupe consistant en un trouble inflammatoire, un trouble allergique, une maladie auto-immune et le rejet d'un greffon.
